# EUROPEAN PATENT APPLICATION

(11) **EP 1 398 025 A1**
(43) Date of publication of application: **17.03.2004**
(21) Application number: 02728072.6
(22) Date of filing: 17.05.2002
(51) Int. Cl.: A61K 9/52, A61K 38/00, A61K 47/04, A61K 48/00, A61P 5/00, A61P 5/06, A61P 5/10, A61P 5/18, A61P 9/00, A61P 9/10, A61P 29/00, A61P 31/00, A61P 35/00, A61P 37/06

(54) **FINE INORGANIC PARTICLES HAVING DRUG INCLUDED THEREIN, METHOD FOR PREPARATION THEREOF AND PHARMACEUTICAL PREPARATION COMPRISING FINE INORGANIC PARTICLES HAVING DRUG INCLUDED THEREIN**

(30) Priority: 28.05.2001 JP 2001158429
(71) Applicant: LTT Bio-Pharma Co., Ltd., Tokyo (JP)
(72) Inventor: MIZUSHIMA, Yutaka, Setagaya-ku, Tokyo 154-0022 (JP); TAKAGI, Yukie, Kawasaki-shi, Kanagawa 214-0035 (JP); HIGAKI, Megumu, Setagaya-ku, Tokyo 156-0057 (JP); IGARASHI, Rie, Kawasaki-shi, Kanagawa 214-0036 (JP); YAMAGUCHI, Yoko, Ashigarakami-gun, Kanagawa 258-0003 (JP); KIMURA, Michio, Kanagawa 253-0003 (JP)
(74) Representative: Rupp, Christian, Dipl.Phys.
(86) International application number: PCT/JP2002/004772
(87) International publication number: WO 2002/096396

(57) **Abstract**

To provide: drug-encapsulating inorganic microparticles in which a method of producing pharmaceutical preparations is simple, which are not stimulative, which are applicable to a great number of potent proteins, potent low-molecular weight compounds and genes, and once applied thereto, enable the potent proteins, potent low-molecular weight compounds and genes to be kept stable, and which give an excellent sustained release effect and a targeting effect to the drugs; a method of manufacturing the same; and pharmaceutical preparations of drug-encapsulating inorganic microparticles.

The above drug-encapsulating inorganic microparticles include: sparingly water-soluble calcium-containing inorganic microparticles; and a biologically active substance encapsulated in the microparticles. The method of manufacturing the drug-encapsulating inorganic microparticles include: (1) preparing an aqueous solution of calcium salt; (2) adding and mixing an aqueous solution of a biologically active substance with the above aqueous solution; and (3) adding and mixing an aqueous solution of carbonate, phosphate, oxalate or urate with the above mixed solution to allow the biologically active substance to be encapsulated in the sparingly water-soluble calcium-containing microparticles. The above pharmaceutical preparations are produced in such a manner as to add pharmaceutically acceptable additives to the drug-encapsulating inorganic microparticles.

## Description

### Technical Field

The present invention relates to sparingly water-soluble calcium-containing inorganic microparticles in which pharmacologically potent proteins, low-molecular weight compounds or genes are encapsulated, a method of manufacturing the same, and pharmaceutical preparations of sparingly water-soluble calcium-containing inorganic microparticles. In more particular, the invention relates to sustained release preparations using sparingly water-soluble calcium-containing inorganic microparticles in which potent proteins, antigens, genes, or potent low-molecular weight compounds are encapsulated, sustained release preparations for targeting and a method of manufacturing the same.

The invention also relates to pharmaceutical preparations of the above microparticles which are encapsulated in the matrices of polylactic acid etc. used in regenerative medicine.

### Background Art

There have been reported techniques in which calcium-containing inorganic substances are used as a carrier for drugs and devised methods in which microcrystals of hydroxyapatite are used as a drug carrier and those carrying anticancer agents on their surface are administered to animals by injection. In addition, there have been proposed sustained release preparations which use porous hydroxyapatite as a drug carrier. However, as for the reports or patents in which sparingly water-soluble calcium-containing inorganic microparticles other than calcium phosphate are used, there have been only patents (Japanese Patent Laid-Open Nos. 07-165613 and 08-027031) which disclose nasal drops using calcium carbonate as a drug carrier.

Further, in the use of calcium-containing inorganic microparticles as a drug carrier, a technique has been employed to attach a drug on the surface of the microparticles, but an approach has never been adopted to encapsulate drugs in the same.

Specifically, a technique has never been employed in which a biologically active substance is allowed to coexist in forming sparingly water-soluble calcium-containing inorganic microparticles and to be encapsulated in the inorganic microparticles at precisely the same time that the microparticles are formed.

In recentyears, with the development of biotechnology, the number of protein drugs has been increased. However, protein drugs can be administered only by injection and many of them have short half-lives. Accordingly, it is required to increase their half-lives by a simple method. ] In addition, it will become an increasingly important subject in the future to direct mainly low-molecular weight drugs, active proteins, vaccines or genes at targets such as macrophage, reticuloendothelial system, neutrophil, vascular endothelical cell, cancer cell, inflammatory site, infectious site, cancer tissue and arteriosclerotic vascular wall.

### Disclosure of the Invention

Accordingly, the object of the present invention is to provide: drug-encapsulating inorganic microparticles in which a method of producing pharmaceutical preparations is simple, which are not stimulative, and which are applicable to a great number of potent proteins, potent low-molecular weight compounds and genes, and once applied thereto, enable the potent proteins, potent low-molecular weight compounds and genes to be kept stable, and which produce an excellent sustained release effect and a targeting effect to the drugs; a method of manufacturing the same; and pharmaceutical preparations of the drug-encapsulating inorganic microparticles.

In order to accomplish the above object, the drug-encapsulating inorganic microparticles of this invention include: sparingly water-soluble calcium-containing inorganic microparticles; and a biologically active substance encapsulated in the microparticles.

The microparticles made up of sparingly water-soluble calcium-containing inorganic microparticles and a biologically active substance encapsulated in the same are simplified to produce, are not stimulative, and enable the biologically active substance used therein, such as potent protein, potent low-molecular weight compound or gene, particularly the potent protein to be kept stable.

The term "to encapsulate" used in this invention means to form sparingly water-soluble calcium-containing inorganic microparticles coexistent with a biologically active substance, so as to bind the biologically active substance to the inside of the resultant inorganic microparticles. The method using this encapsulation technique gives the effects of increasing the drug-encapsulation rate and slowing down the drug elution from the microparticles, compared with the method in which first inorganic microparticles are formed and then a drug is admixed with the microparticles so that it is carried on the same.

The potent proteins include, for example, erythropoietine (EPO), granulocyte colony-stimulating factor (G-CSF), granulocyte macrophage colony-stimulating factor (GM-CSF), thrombopoietine, interferon α, interferon β, interferon γ, urokinase, tissue plasminogen activator (t-PA), interleukin-11 (IL-11), Enbrel, fibroblast growth factor (FGF), epidermal growth factor (EGF), hepatocyte growth factor (HGF), brain-derived neurotrophic factor (BDNF), nerve growth factor (NGF), leptin, neutrophin-3 (NT-3), superoxide dismutase (SOD), insulin, human growth hormone, antibody and antigen. Of the above proteins, EPO, G-CSF, interferon α, FGF, EGF, HGF, BDNF, NGF, leptin, NT-3 are particularly preferable.

Preferably, the content of the biologically active substance is 0.0001 to 10% by weight of that of the above sparingly water-soluble calcium-containing inorganic matter. Further, the biologically active substance should be a drug capable of binding to calcium, and generally such a substance negatively charged is preferable because calcium is positively charged.

The potent low-molecular weight compounds include, for example, non-steroidal anti-inflammatory agents, anti-inflammatory agents such as hydrocortisones, antimicrobial agents, anticancer agents, vasoactive agents such as prostaglandin, antiarteriosclerosis agents, immunosuppressive agents, calcitonin, luteinizing hormone releasing hormone (LHRH) derivative, other pituitary peptide hormones, vancomycin, teicoplanin, and parathyroid hormone (PTH). Particularly, antimicrobial agents such as antibacterial agents and antifungal agents, anti-inflammatory agents, anticancer agents, and vasoactive agents are preferable. When the low-molecular weight drugs have a poor capacity of binding to calcium, the drugs should be used with their residues covalently bound to a compound capable of highly binding to calcium, such as phosphoric acid, by esterification.

Preferable sparingly water-soluble calcium-containing inorganic matter includes, for example, calcium carbonate, calcium phosphate (e.g. apatite, hydroxyapatite), calcium oxalate and calcium urate.

The present inventors directed their attention to the point that the calcium-containing inorganic matter was sparingly water-soluble and in the form of microparticles, and then they aimed at targeting effect and sustained release effect for drugs by allowing drugs to be encapsulated in the calcium-containing inorganic microparticles and administering the drug-encapsulating microparticles by injection so that the drugs are targeted at focuses and released little by little in the body. Specifically, at the sites of inflammation and infection, the cancer tissue, the arteriosclerotic vessel wall, etc., there exist gaps several tens to several hundred nanometers in size in the walls of their blood vessels, and of the microparticles of the present invention, those 10 nm to 1,000 nm in size, preferably 10 nm to 500 nm in size will pass through the gaps and accumulate at such focuses. As a result, the targeting effect for drugs can be produced, and moreover, since inflammatory cells, such as macrophage, and cancer cells phagocytize drugs, double targeting effect can also be produced, besides the sustained release effect.

When the drug-encapsulating microparticles are 100 nm to 200 µm in diameter, they are useful as sustained release preparations for subcutaneous and intramuscular injections.

The pharmaceutical preparations of the drug-encapsulating microparticles of the present invention exhibit a good performance in sustained release of growth factors used in regenerative medicine, which has remarkably progressed in recent years. When using the preparations in regenerative medicine, preferably a little larger microparticles are used as they are or in a state in which they are encapsulated in matrices of polylactic acid (PLA).

The final preparations are produced by adding pharmaceutically acceptable additives, in particular, proteins such as human serum albumin (HSA), acid mucopolysaccharides, polylactic glycolic acid (PLGA), polylactic acid, surfactants, dispersants such as mannitol, stabilizers and antiseptics to the resultant drug-encapsulating inorganic microparticles and drying or freeze-drying the same. The resultant final preparations are suspended in water or buffer solutions so that the solutions become isotonic with body fluid, and then, administered to humans. In order to prevent pain etc. or improve the dispersion of the preparations, the final preparations can be used in the form of a suspension in hyaluronic acid. Or the suspensions of the drug-encapsulating inorganic microparticles to which dispersants etc. have been added can also be used as final preparations.

The final preparations described above are in the form suitable for subcutaneous, intramuscular and intravascular injections.

A method of manufacturing the drug-encapsulating inorganic microparticles of this invention includes: (1) preparing an aqueous solution of calcium salt such as calcium chloride, calcium bromide or calcium acetate; (2) adding and mixing an aqueous solution of a biologically active substance with the above solution; and (3) adding and mixing an aqueous solution of carbonate such as sodium carbonate or potassium carbonate, phosphate such as sodium phosphate, sodium hydrogen phosphate or potassium phosphate, oxalate such as sodium oxalate or potassium oxalate, or urate such as sodium urate or potassium urate with the above solution to allow the biologically active substance to be encapsulated in sparingly water-soluble calcium-containing inorganic microparticles.

Another method of manufacturing the drug-encapsulating inorganic microparticles of this invention includes: (1) preparing an aqueous solution of carbonate, phosphate, oxalate or urate; (2) adding and mixing an aqueous solution of a biologically active substance with the above solution; and (3) adding and mixing an aqueous solution of calcium salt such as calcium chloride, calcium bromide or calcium acetate with the above solution to allow the biologically active substance to be encapsulated in sparingly water-soluble calcium-containing inorganic microparticles.

In order to prevent the aggregation of the microparticles, during their production, of sparingly water-soluble calcium-containing inorganic matter in which a biologically active substance has been encapsulated, preferably protein, acid mucopolysaccharide, surfactant and mannitol are added to the reaction solution. Further, in order to prevent the aggregation of the microparticles, in the body, of sparingly water-soluble calcium-containing inorganic matter in which a biologically active substance has been encapsulated and avoid the microparticles being subjected to phagocytosis in the body, preferably protein, acid mucopolysaccharide, polylactic glycolic acid and polylactic acid are added to the drug-encapsulating inorganic microparticles.

When two or more kinds of inorganic matter are admixed in the manufacturing of drug-encapsulating inorganic microparticles, preferably they are admixed with stirring at about pH 7. Altering the concentration of the inorganic matter, the concentration of the drug, the stirring speed, and the operating time and temperature control the size of the particles. Ordinary stirring using a stirrer only produces fine grains 1 µm to 100 µm in size, but if stirring power is enhanced using Vortex, Polytron or ultrasonic, fine grains 10 nm to 100 µm in size can also be produced.

Desirably, the aqueous solution is kept as neutral as possible, the ion strength is kept as low as possible, and a buffer solution which does not bind to calcium is used.

### Brief Description of the Drawings

Figure 1 is a graph showing the amounts of EPO in the compositions of example 1 and test example 1;
Figure 2 is a graph showing the amounts of G-CSF in the compositions of example 2 and test example 2;
Figure 3 is a graph showing the amounts of HyC (Phos.) in the compositions of example 3 and test example 3;
Figure 4 is a graph showing the release characteristics of G-CSF released from the composition of example 4, along with the stability of G-CSF in a buffer solution as a contrast;
Figure 5 is a graph showing the change of blood EPO concentration after giving the preparation of example 5 into a mouse by intramuscular injection;
Figure 6 is a graph showing the significant transfer, compared with the control, of the preparation of example 6 into a mouse spleen after intravenously giving the preparation into the mouse; and
Figure 7 is an observed view of the preparation of example 7 being taken up by macrophages.

### Best Mode for Carrying Out the Invention

In the following the examples and test examples of the present invention will be described.

### (Example 1)

Six hundred fifty µl of 5M CaCl₂ (Wako) was admixed with 125 µl of 1 mg/ml EPO (Chugai Pharmaceutical) with stirring. Then 2.5 ml of 1M Na₂CO₃ (Wako) was added thereto and stirred for 10 minutes to form CaCO₃ particles while allowing the EPO to be encapsulated in the particles. Five ml of H₂O was added to the particles, and the mixture was centrifuged at 2,000 rpm for 5 min to remove the supernatant. To the resultant precipitate, 6.25 ml of H₂O was added, and the mixture was dispensed in 1 ml portions into four 1.5 ml test tubes. The four test tubes and contents were centrifuged at 2,000 rpm for 5 min to remove the supernatant. The contents of two of the four tubes were used for ELISA to quantitatively determine the EPO having been encapsulated in the CaCO₃ particles. For the rest two of the tubes and contents, 0.9 ml of 1M Na₂CO₃ was added thereto, and the mixture was stirred and allowed to stand still to separate the EPO binding to the surface of the CaCO₃ particles. After left stand still for 10 min, the mixture was centrifuged to remove the supernatant. The same operation was repeated once, and the EPO having been separated by Na₂CO₃ treatment was removed by centrifugation. The resultant precipitate was used for ELISA. The EPO having been encapsulated in the CaCO₃ particles was separated from the CaCO₃ particles by dissolving the same with hydrochloric acid and quantitatively determined by ELISA.

### (Test Example 1)

A sample was prepared in such a manner as to first admix 650 µl of 5M CaCl₂ with 2.5 ml of 1M Na₂CO₃ to form CaCO₃ particles, then the CaCO₃ particles was washed with H₂O, and 125 µl of 1 mg/ml EPO was added thereto. Five ml of H₂O was added to the sample, and the mixture was centrifuged at 2,000 rpm for 5 min to remove the supernatant. Then the same operation as in example 1 was performed to quantitatively determine the EPO existing on the surface of the CaCO₃ particles.

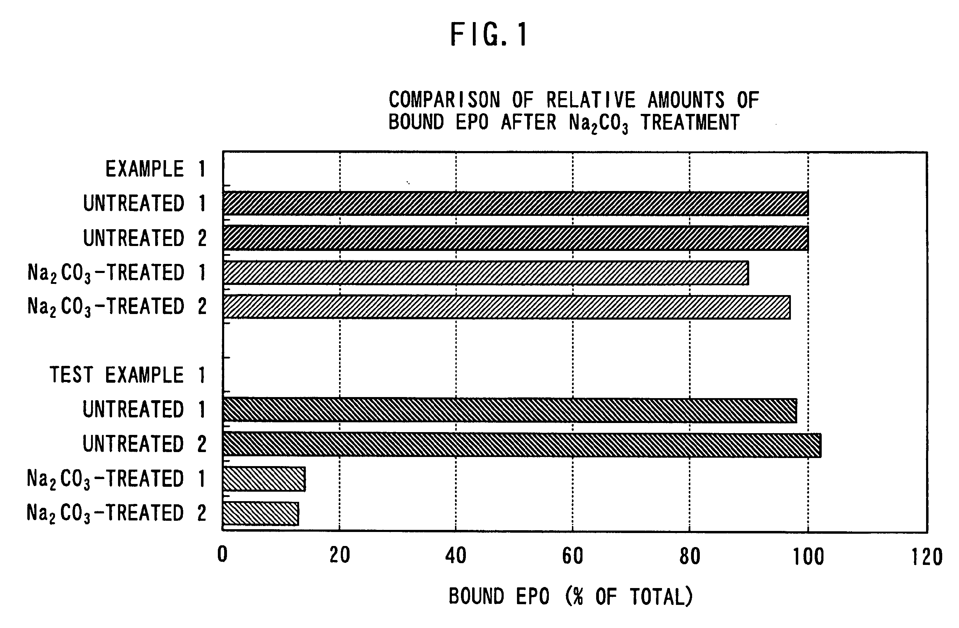

The result revealed, as shown in Figure 1, that unlike the sample of test example 1, in which EPO was bound to the surface of the CaCO₃ particles, in the microparticle sample obtained by the encapsulation method of example 1 based on the present invention, EPO was hardly separated from the CaCO₃ particles by Na₂CO₃ treatment and almost the entire EPO was encapsulated in the particles.

### (Example 2)

Six hundred fifty µl of 5M CaCl₂ was admixed with 250 µl of 500 µg/ml G-CSF (Chugai Pharmaceutical) with stirring. Then 2.5 ml of 1M Na₂CO₃ was added thereto and stirred for 10 minutes to prepare CaCO₃ particles. Five ml of H₂O was added to the particles, and the mixture was centrifuged at 2 , 000 rpm for 5 min to remove the supernatant. To the resultant precipitate, 6.25 ml of H₂O was added, and the mixture was dispensed in 1 ml portions into four 1.5 ml test tubes. The four test tubes and contents were centrifuged at 2,000 rpm for 5 min to remove the supernatant. The contents of two of the four tubes were used for ELISA to quantitatively determine the G-CSF having been encapsulated in the CaCO₃ particles. For the rest two of the tubes and contents, 0.9 ml of 1M Na₂CO₃ was added thereto, and the mixture was stirred and allowed to stand still to separate the G-CSF binding to the surface of the CaCO₃ particles. After left stand still for 10 min, the mixture was centrifuged to remove the supernatant. The same operation was repeated once, and the G-CSF having been separated by Na₂CO₃ treatment was removed by centrifugation. The resultant precipitate was used for ELISA. The G-CSF having been encapsulated in the CaCO₃ particles was separated from the CaCO₃ particles by dissolving the same with hydrochloric acid and quantitatively determined by G-CSF ELISA (IBL).

### (Test Example 2)

A sample was prepared in such a manner as to first admix 650 µl of 5M CaCl₂ with 2.5 ml of 1M Na₂CO₃ to form CaCO₃ particles, then the CaCO₃ particles was washed with H₂O, and 250 µl of 500 µg/ml G-CSF was added thereto. Five ml of H₂O was added to the particles, and the mixture was centrifuged at 2,000 rpm for 5 min to remove G-CSF in the supernatant. Then the same operation as in example 2 was performed to quantitatively determine the G-CSF existing on the surface of the CaCO₃ particles.

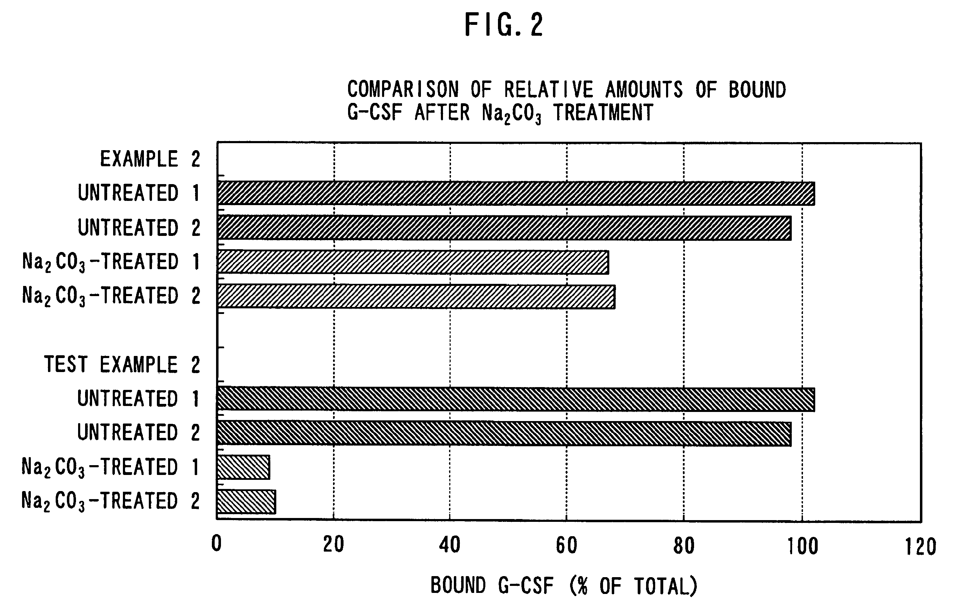

The result proved, as shown in Figure 2, that unlike the sample of test example 2 in which G-CSF was bound to the surface of the CaCO₃ particles, in the sample obtained by the encapsulation method of example 2, G-CSF was a little separated from the CaCO₃ particles by Na₂CO₃ treatment, but a significant amount of G-CSF was encapsulated in the particles.

### (Example 3).

Six hundred fifty µl of 5M CaCl₂ was admixed with 375 µl of a preparation containing 5% hydrocortisone phosphate [HyC (Phos.)] (water-soluble Hydrocortone, Banyu Pharmaceutical) with stirring using Vortex. Then 2.5 ml of 1M Na₂CO₃ was added and stirred for 10 minutes to prepare CaCO₃ particles. Five ml of H₂O was added to the particles, and the mixture was centrifuged at 2,000 rpm for 5 min to remove the HyC (Phos.) not encapsulated in the CaCO₃ particles. Part of the resultant CaCO₃ particles was used for ELISA. On the other hand, 6.25 ml of H₂O was added to the above precipitate, and 1 ml of the mixture was taken into a 1.5 ml test tube. Hydrochloric acid was added to the mixture to dissolve the CaCO₃ particles entirely and the mixture was used for ELISA. Before carrying out ELISA, the mixture was admixed with a mouse liver homogenate in the ratio of 1 : 1, and the admixture was incubated at 37°C for 2 hours to hydrolyze HyC (Phos.) to give free HyC and used for the assay. The liver homogenate was prepared by taking a liver from a mouse, adding 5 ml of H₂O per individual liver and homogenizing the mixture using Polytron, centrifuging the homogenized mixture at 15,000 rpm for 5 min, and collecting the supernatant as the liver homogenate.

### (Test Example 3)

A sample was prepared in such a manner as to first admix 650 µl of 5M CaCl₂ with 2.5 ml of 1M Na₂CO₃ to prepare CaCO₃ particles, then the CaCO₃ particles was washed with H₂O, and 375 µl of HyC (Phos.)was added thereto. Five ml of H₂O was added to the mixture, and the mixture was centrifuged at 2,000 rpm for 5 min to remove HyC (Phos.) not bound to the CaCO₃ particles. Then the same operation as in example 3 was performed to quantitatively determine HyC existing in or on the CaCO₃ particles.

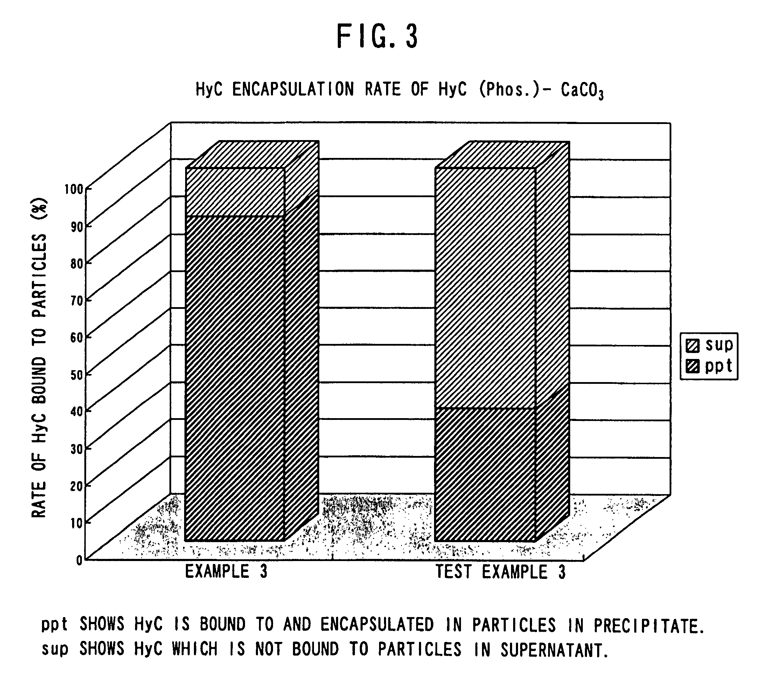

The result was, as shown in Figure 3, that the amount of HyC which was bound to or encapsulated in the CaCO₃ particles was 70% or more of the total amount of HyC in the suspension for the sample of example 3, and about 20% for the sample of test example 3 and the result proved that a low-molecular weight drug was also encapsulated in the CaCO₃ particles.

### (Example 4)

Six hundred fifty µl of 5M CaCl₂ was admixed with 250 µl of 500 µg/ml G-CSF with stirring. Then 2.5 ml of 1M Na₂CO₃ was added and stirred for 10 minutes to prepare CaCO₃ particles. Five ml of H₂O was added to the particles, and the mixture was centrifuged at 2,000 rpm for 5 min to remove the supernatant. To the resultant precipitate, 12.5 ml of H₂O was added, and 1 ml of the mixture was taken into a 2.0 ml test tube. The test tube and content was centrifuged at 2,000 rpm for 5 min to remove the supernatant which was used for ELISA. To the resultant precipitate, 1 ml of 1% BSA/Tris - HCl (pH 7.2) was added and shaken at room temperature. The mixture was centrifuged every 24 hours at 2,000 rpm for 5 min to collect the supernatant and to the resultant precipitate 1ml of 1% BSA/Tris - HCl (pH 7.2) was added again, and this operation was continued for 7 days. The last precipitate was dissolved with hydrochloric acid and used for ELISA. G-CSF was assayed using ELISA Kit manufactured by IBL Co. and the total (cumulative) amount of G-CSF released from the G-CSF-encapsulating CaCO₃ preparation was shown. As a control, both 30 µl of 100 µg /ml G-CSF and 5 ml of 1% BSA/Tris - HCl (pH 7.2) were added at the same time to the precipitate and admixed with each other, and the admixture was shaken at room temperature. A part of the mixture was collected every 24 hours, and the amount of G-CSF was determined by ELISA to examine the stability of G-CSF at room temperature.

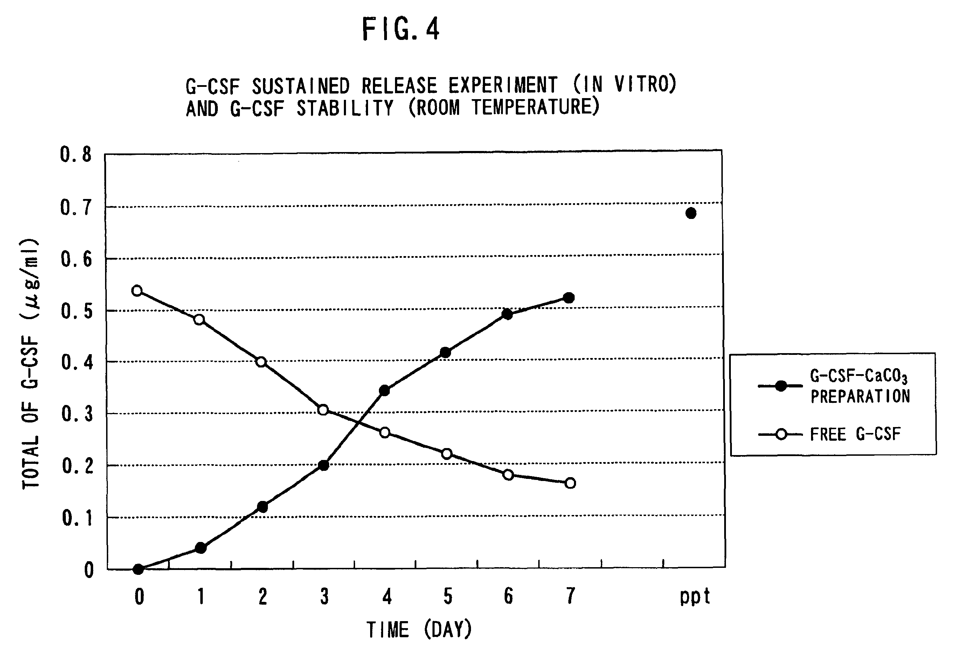

The results are shown in Figure 4. The G-CSF release test revealed that the G-CSF encapsulated in the CaCO₃ particles was released therefrom little by little over 7 days. The total amount of G-CSF including the amount of G-CSF in the precipitate after 7 days was 0.7 µg, which was considerably smaller compared with the amount of G-CSF used (10 µg). This is possibly because the G-CSF having been eluted in the buffer solution was unstable. The unstableness of the G-CSF in the buffer solution is also apparent from the deactivation curve (open circle, dotted line) when the solution of free G-CSF was left stand at room temperature. It was shown that the G-CSF in CaCO₃ particles was much more stable than that in a solution.

### (Example 5)

Six hundred fifty µl of 5M CaCl₂ was admixed with 125 µl of 1 mg/ml EPO with stirring. Then 2.5 ml of 1M Na₂CO₃ was added and stirred for 10 minutes to prepare CaCO₃ particles. Five milliliters of H₂O was added to the particles, and the mixture was centrifuged at 2,000 rpm for 5 min to remove the supernatant. To the resultant precipitate, 3 ml of H₂O was added, and 1 ml of the mixture was taken into a 1.5 ml test tube. The test tube and content were centrifuged at 2,000 rpm for 5 min to remove the supernatant entirely. A sample was prepared by adding 0.3 ml of 2% chondroitin sulfate A sodium (CS-A, Wako) and 2.1 ml of 5% Mannitol (Wako) to the resultant precipitate and stirring the mixture. As a control, a sample was also prepared by admixing 30 µl of 1 mg/ml EPO with 1.77 ml of 5% Mannitol. These samples were given to 5-month old male C3H/He mice by intramuscular injection of 200 µl, and blood samples were collected from their orbit 4 hours, 1 day, 2, 3 and 4 days after the administration, respectively, to quantitatively determine the blood EPO concentrations by ELISA.

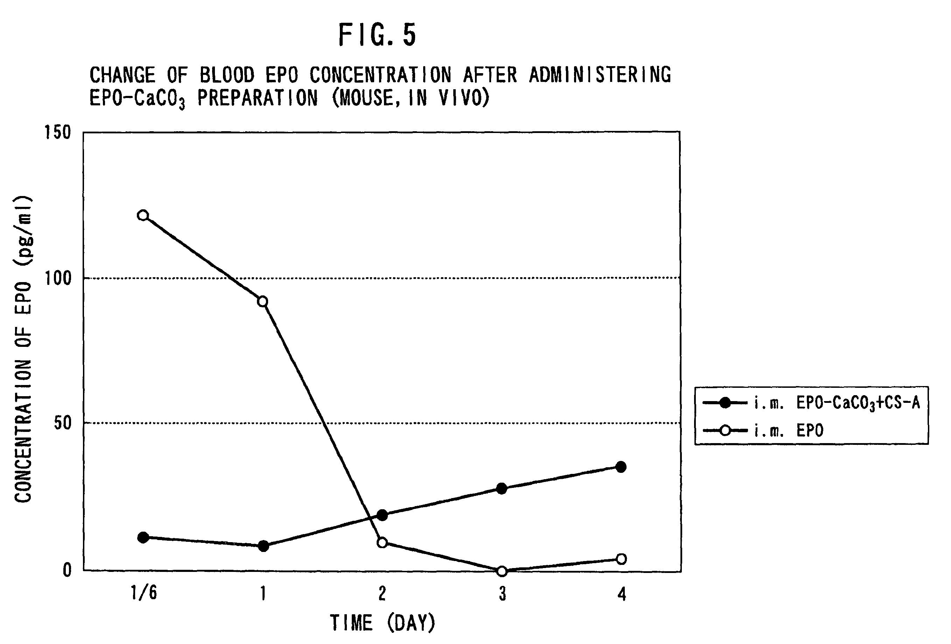

The results are shown in Figure 5. When intramuscularly giving the EPO-CaCO₃ preparation on which CS-A was made to act to prevent the particle's aggregation or adhesion to tissues, sustained release effect can be produced in vivo.

### (Example 6)

Six hundred fifty µl of 5M CaCl₂ was admixed with 375 µl of preparation containing 5% HyC (Phos.) with stirring. Then 2.5 ml of 1M Na₂CO₃ was added and stirred to prepare CaCO₃ particles. To prevent adhesion among the prepared CaCO₃ particles, 3.525 ml of 2% CS-A was added and stirred for 10 min. Five ml of H₂O was added to the mixture, and the mixture was centrifuged at 2,000 rpm for 5 min to remove the HyC (Phos.) not bound to the CaCO₃ particles. To the resultant precipitate 1.397 ml of 1% CS-A/5% Mannitol was added, which was used to administer to a rat. As a control, 300 µl of HyC (Phos.) and 818 µl of 0.1% Tween 80/0.5% BSA/5% Mannitol were admixed and prepared, 500 µl of which was given to a 10-week old Wistar rat by intravenous injection. Blood samples were collected from the Wistar rat 10 min and 1 hour after the administration and their spleens 48 hours after the administration. In order to decompose the HyC (Phos.) remaining as phosphate into HyC, mouse liver homogenate was made to act on the collected blood samples at 37°C for 2 hours. After fully homogenizing the collected spleens and disrupting the cells, mouse liver homogenate was made to act thereon. Then the homogenized spleens were extracted with DMSO and quantitatively determined by ELISA. The amount of HyC was obtained by subtracting the amount of HyC of rat to which the preparation was not administered. In ELISA, Cortisol EIA (IBL) was used.

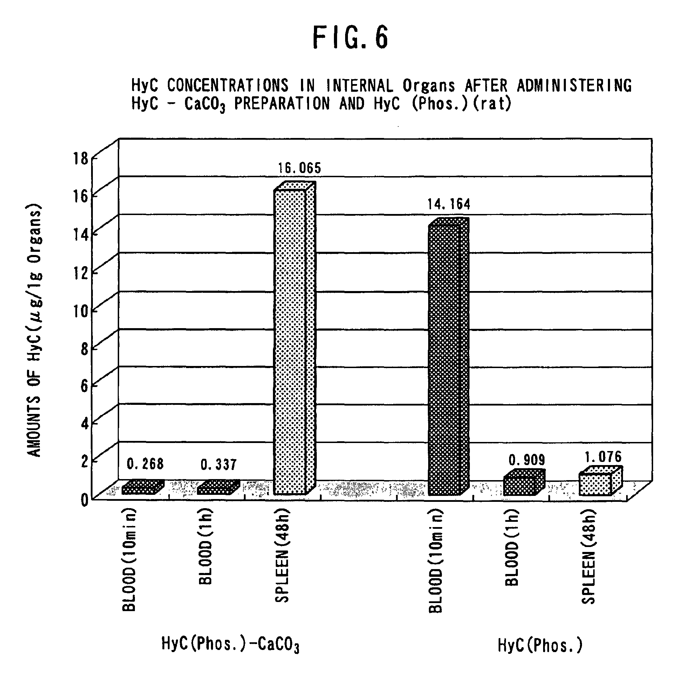

As shown in Figure 6, it was revealed that in the preparation of CaCO₃ particles, HyC was targeted at the spleen where there existed gaps in the vascular walls, like inflammation nests or cancer tissues.

### (Example 7)

Six hundred fifty µl of 5M CaCl₂ was admixed with 375 µl of preparation containing 5% HyC (Phos.) with stirring. Then 2.5 ml of 1M Na₂CO₃ was added and stirred to prepare CaCO₃ particles. To prevent adhesion among the prepared CaCO₃ particles, 3.525 ml of 2% CS-A was added and stirred for 10 min. Five ml of H₂O was added to the mixture, and the mixture was centrifuged at 2,000 rpm for 5 min to remove the HyC (Phos.) not bound to the CaCO₃ particles. To the resultant precipitate 12.5 ml of 1% CS-A/5% Mannitol was added to prepare a sample. 2.0 ml of 10% proteose peptone was administered into the abdominal cavity of a mouse by injection, andafter3days, exudate peritoneal macrophage was collected. The macrophage was prepared to be 5 × 10⁵ cells/ml, and 0.1 ml of the cell suspension was put on a 24-wells plate with a cover glass and incubated to attach the macrophage thereon. After removing the cells not having been attached on the cover glass and replacing the culture, 0.375 ml of HyC (Phos.)-encapsulating CaCO₃ was added and incubated at 37°C. After 24-hour incubation, the cover glass was taken, air-dried, and stainedby Giemsa staining method so that the macrophages' phagocytosis for the CaCO₃ particles was observed.

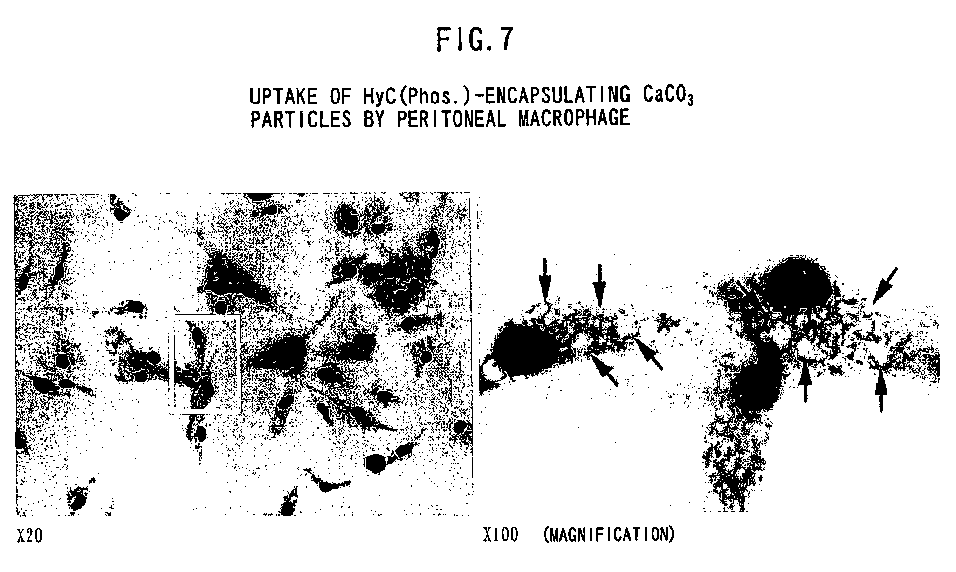

The result is shown in Figure 7, and the image was observed in which HyC (Phos.)-encapsulating CaCO₃ particles were phagocytized by the macrophages.

### (Example 8)

2.6 ml of 5M CaCl₂ was admixed with 1 ml of 500 µg/ml G-CSF with stirring. Then 10 ml of 1M Na₂CO₃ was added and stirred for 10 minutes to prepare CaCO₃ particles. Twenty ml of H₂O was added to the particles, and the mixture was centrifuged at 2,000 rpm for 5 min to remove the G-CSF in the supernatant. To the resultant precipitate, 3 ml of H₂O was added, and the mixture was taken into a vial. The mixture was then freeze-dried into powder. Fifty mg of G-CSF-including CaCO₃ particles, 2.61 g of PLGA and 3 ml of dichloromethane were admixedwith each other. This mixture was admixed with a 0.1% polyvinyl alcohol/0.7% zinc acetate solution with stirring. After 3-hour stirring, the mixture was centrifuged at 1,000 rpm to obtain a precipitate. The precipitate was washed with H₂O and passed though a 250 µm filter, and 0.7 µl of 20% mannitol was added to the filtrate. Then the mixture was freeze-dried to give a sustained release preparation.

## Claims

1. Drug-encapsulating inorganic microparticles, comprising sparingly water-soluble calcium-containing inorganic microparticles and a biologically active substance encapsulated therein.

2. Drug-encapsulating inorganic microparticles according to claim 1, **characterized in that** the biologically active substance is a potent protein, a potent low-molecular weight compound or a gene.

3. Drug-encapsulating inorganic microparticles according to claim 1, **characterized in that** the content of the biologically active substance is 0.0001 to 10% by weight of that of the sparingly water-soluble calcium-containing inorganic microparticles.

4. Drug-encapsulating inorganic microparticles according to claim 1, **characterized in that** the potent protein is EPO, G-CSF, GM-CSF, thrombopoietine, interferon α, interferon β, interferon γ, urokinase, t-PA, IL-11, Enbrel, FGF, EGF, HGF, BDNF, NGF, leptin, NT-3, SOD, insulin, human growth hormone, antibody or antigen.

5. Drug-encapsulating inorganic microparticles according to claim 1, **characterized in that** the potent low-molecular weight compound is non-steroidal anti-inflammatory agent, an anti-inflammatory agent such as hydrocortisones, an antimicrobial agent, an anticancer agent, an vasoactive agent such as prostaglandin, an anti-arteriosclerosis agent, an immunosuppressive agent, calcitonin, a LHRH derivative, other pituitary peptide hormone, vancomycin, teicoplanin or PTH.

6. Drug-encapsulating inorganic microparticles according to claim 1, **characterized in that** the sparingly water-soluble calcium-containing inorganic matter is calcium carbonate, calcium phosphate, calcium oxalate or calcium urate.

7. Drug-encapsulating inorganic microparticles according to claim 1, **characterized in that** the sparingly water-soluble calcium-containing inorganic microparticles are 100 nm to 200 µm in diameter.

8. Drug-encapsulating inorganic microparticles according to claim 1, **characterized in that** the sparingly water-soluble calcium-containing inorganic microparticles are 10 nm to 1,000 nm in diameter.

9. Pharmaceutical preparations of drug-encapsulating inorganic microparticles, **characterized in that** they comprise the drug-encapsulating inorganic microparticles according to claim 1 and pharmaceutically acceptable additives added thereto.

10. The pharmaceutical preparations of drug-encapsulating inorganic microparticles according to claim 9, **characterized in that** the pharmaceutically acceptable additives are protein, acid mucopolysaccharide, polylactic glycolic acid, polylactic acid, surfactant, mannitol, antiseptics and stabilizer.

11. The pharmaceutical preparations of drug-encapsulating inorganic microparticles according to claim 9 or 10, **characterized in that** the pharmaceutical preparations of drug-encapsulating inorganic microparticles according to claim 9 are in the form suitable for subcutaneous, intramuscular or intravascular injections.

12. A method of manufacturing drug-encapsulating inorganic microparticles, **characterized in that** it comprises: (1) preparing an aqueous solution of calcium salt; (2) adding and mixing an aqueous solution of a biologically active substance with said aqueous solution; and (3) adding andmixing an aqueous solution of carbonate, phosphate, oxalate or urate with said mixed solution to allow the biologically active substance to be encapsulated in the sparingly water-soluble calcium-containing microparticles.

13. A method of manufacturing drug-encapsulating inorganic microparticles, **characterized in that** it comprises: (1) preparing an aqueous solution of carbonate, phosphate, oxalate or urate; (2) adding and mixing an aqueous solution of a biologically active substance with said aqueous solution; and (3) adding andmixing an aqueous solution of calcium salt with said mixed solution to allow the biologically active substance to be encapsulated in the sparingly water-soluble calcium-containing microparticles.

14. The method of manufacturing drug-encapsulating inorganic microparticles according to claim 12 or 13, **characterized in that** protein, acid mucopolysaccharide, surfactant or mannitol are added to the reaction solution so as to prevent the aggregation, during their production, of the sparingly water-soluble calcium-containing inorganic microparticles in which the biologically active substance has been encapsulated.
